# EUROPEAN PATENT SPECIFICATION

(11) **EP 0 577 894 B1**
(45) Date of publication and mention of the grant of the patent: **07.01.1998**
(21) Application number: 92310032.5
(22) Date of filing: 02.11.1992
(51) Int. Cl.: C07K 14/155, C12N 15/48, A61K 39/21

(54) **Design, construction and expression of chimeric proteins for development of vaccines and diagnostic reagents**
Entwurf, Konstruktion und Expression von chimärischen Proteinen zur Entwicklung von Impfstoffen und diagnostischen Reagenzien
Conception, construction et expression de protéines chimériques pour le développement de vaccins et de réactifs diagnostiques

(30) Priority: 17.06.1992 KR 1049392
(43) Date of publication of application: 12.01.1994
(73) Proprietor: KOREA GREEN CROSS CORPORATION, Yongin-Kun, Kyongki-Do 449-900 (KR)
(72) Inventor: Kang, Chil-Yong, London, Ontario N6G 4X9 (CA); Luo, Lizhong, London, Ontario N5Y 4X9 (CA)
(74) Representative: Griffin, Kenneth David

(56) References cited:
- WO-A-87/05399
- CHEMICAL ABSTRACTS, vol. 118, no. 3, January 18, 1993, Columbus, Ohio, USA LUO, LIZHONG et al. "Chimeric gag-V3 virus-like particles of human immunodeficiency virus induce virus-neutrali- zing antibodies.", page 536, column 2, abstract- - no. 20 650v
- CHEMICAL ABSTRACTS, vol. 116, no. 5, February 3, 1992, Columbus, Ohio, USA TRAUNECKER, ANDRE et al. "Bispecific single chain molecules (Janusins) target cytotoxic lymphocytes on HIV infected cells.", page 606, column 2, abstract- - no. 39 564a
- CHEMICAL ABSTRACTS, vol. 113, no. 15, October 8, 1990, Columbus, Ohio, USA WELDON, ROBERT A., Jr. et al. "Incorporation of chimeric gag protein into retroviral particles.", page 198, column 1, abstract- no. 127 642t

## Description

### BACKGROUND OF THE INVENTION

### 1. Field of the Invention

The present invention relates to construction of chimeric proteins useful for AIDS vaccine, for development of diagnostic reagents and a process for production thereof. More particularly, the present invention relates to *gag* chimeric protein of HIV expressed in the recombinant baculovirus infected insect cells, and a process for production thereof.

### 2. Description of the Prior Art

Type 1 and 2 of the human immunodeficiency viruses(HIV) are recognized as the etiologic agents for acquired immunodeficiency syndrome(AIDS). The vaccine against the said viruses is an ideal way of preventing the said syndrome from infection with HIV, therefore much researches have been focused on molecular biological analyses of structures and functions of HIV. The main virion structural proteins of HIV are derived from the three structural genes known as *gag, pol, env.* The genome of many different isolates of HIVs have been completely sequenced and amino acids sequences have been deduced from the cloned proviral DNA sequences. The envelope gene of HIV codes for a glycoprotein precursor with of molecular weight 160,000(gp160). The precursor gp160 in virus infected cells is processed(or cleaved) to produce envelope glycoprotein gp120 and gp41. The envelope glycoprotein gp120 of HIV has been the major target for developing a candidate vaccine against AIDS. gp120 recognizes the cellular receptor(CD4) on helper T lymphocytes and carries the V3 loop domain that induces neutralizing antibodies (Science 234, 1392-1395, 1986; Proc. Natl. Acad. Science, USA 83, 7023-7027).

The V3 loop represents the third hypervariable region of HIV-1 gpl20(amino acid residues 308-331) which contains not only a major immunodominant neutralizing epitope but also the epitopes for antigen-dependent cellular cytotoxicity(ADCC) and cytotoxic T-lymphocyte(CTL) recognition. Although the majority of the amino acids in the V3 loop are variable among different strains of HIV, a G-P-G-R motif at the tip of the loop is conserved(Science 249, 932-935, 1990).

Recently Huang et al. and Björling et al. demonstrated that the principal neutralization domain of the envelope glycoprotein of HIV-2 is also located in the region corresponding to the hypervariable motif in the V3 loop of HIV-1 gp120. The CD4-binding region, which is located within C-terminal third of HIV-1 gp120(amino acid residues 397-439) plays an essential role in infectivity of HIV. This region also seems to be weakly immunogenic because it forms a pocket which is not accessible to immune system, thus high-titre neutralizing antibody against this region is not presently available.

Chem Abs 113 (1990) No. 127642 describes the synthesis of a chimeric gene by coupling RSV gag to cytochrome and expressing them.

Abstract M.A. 68 from the VII International Conference on AIDS, Florence 1991 describes how chimeric oligopeptides were prepared by linking either a gp 20 or a gp 41 B-cell epitope to either the N- or C- terminus of a p 24 T-cell epitope, p 24E. The immunogenicity of the chimeric oligopeptides was then compared. The authors found that the immunogenicity of the major neutralizing epitope (V3 loop, residues 311-327) was higher when the peptide was linked to the C- rather than the N- terminus of p 24E, whereas the B- cell epitope (V3 loop, resilues 311-327) was higher when linked to the C- terminus of the T-cell epitope to elicit an anti-BE3 antibody response.

### SUMMARY OF THE INVENTION

gag-env chimeric proteins can be obtained by linking gag of HIV to env to form the chimeric gene, inserting the obtained chimeric gene into the DNA of baculovirus, infecting insect cells or insects with the resultant recombinant virus, culturing it and purifying the obtained chimeric protein. Such chimeric proteins are useful for an AIDS vaccine.

According to one aspect of the invention there is provided a recombinant gag-env chimeric polypeptide particle of HIV comprising an HIV-2 gag polypeptide selected from the group consisting of HIV-2 gag polypeptides which extend from the N-terminal amino acid of gag to a minimum of amino acid 376 and a maximum of amino acid 425 and wherein said gag polypeptides have a proline at amino acid 373, and an env polypeptide from HIV comprising the V3 loop domain of gp 120, the env polypeptide being linked to the C-terminus of the gag polypeptide

### BRIEF DESCRIPTION OF THE DRAWINGS

The invention will be described in detail with reference to the accompanying drawings in which:

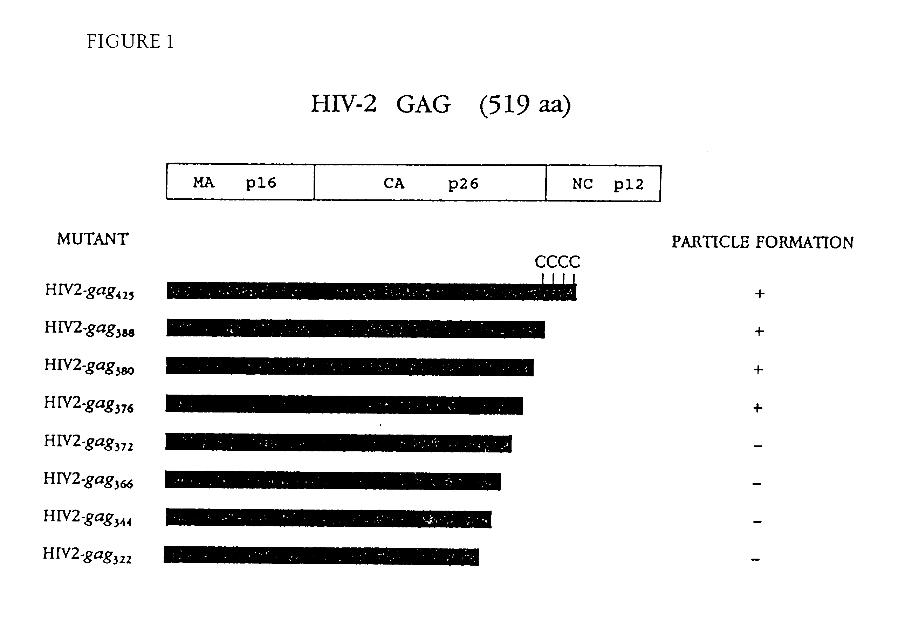

Fig. 1 shows deletion mutants of HIV-2 *gag* gene and particle formation. A series of deletion mutants of HIV-2 gag gene were constructed by PCR with specific primers and the size of the deletion mutant are indicated by the amino acid numbers which are retained. Deletion mutants of HIV-2-*gag*₄₂₅, -*gag*₃₈₈,-*gag*₃₈₀, and -*gag*_{*3*}₇₆ formed virus-like particles and released into culture medium whereas deletion mutants HIV1-*gag*₃₇₂, -*gag*₃₆₆, -*gag*₃₄₄, -*gag*₃₂₂ did not. Polypeptide domains of HIV-2 *gag* protein, p16 MA(matrix protein), p26 CA(capsid protein) and p12 NC(nucleic acid binding protein) are shown on the top of diagram. C on HIV-*gag*₄₂₅ denoted cysteine which represent the zinc finger domain.

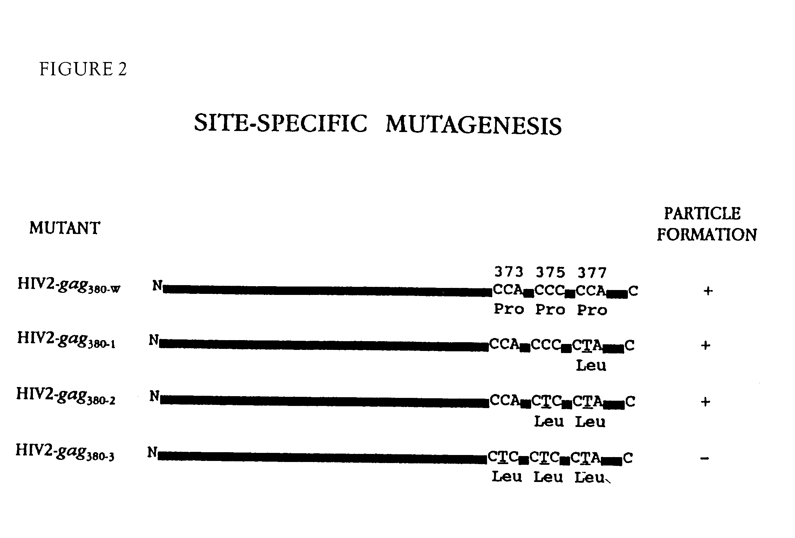

Fig. 2 shows site-specific mutants of HIV-2-gag_{380-W} gene with particle formation. Three prolines located at amino acid position 373, 375 and 377 at the C-terminus of *gag* mutant, HIV-2-*gag*_{380-W}, was substituted with 1,2 or 3 leucine residues by site-directed mutagenesis using PCR and expressed in SF9 cells. The subsitution of up to two prolines did not affect the *gag* particle formation, whereas changing of all three prolines resulted in the failing of particle formation as examined by electron microscopy.

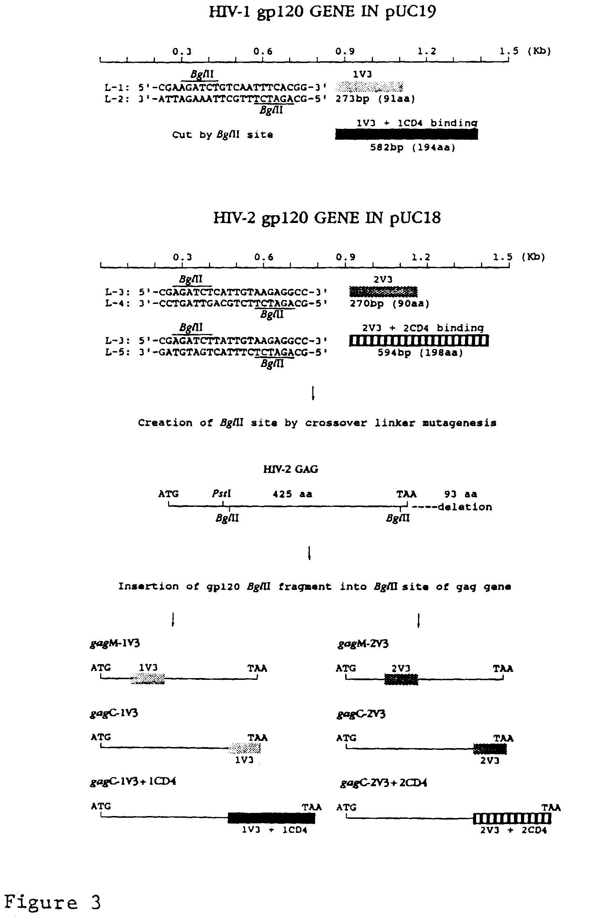

Fig. 3 shows construction of chimeric *gag-env* genes. *Bgl*II fragments containing the V3(amino acid positions 273-363) or V3+CD4BD(amino acid position 294-491) from HIV-2 gp120 were amplified by PCR. The V3+CD4BD sequences of HIV-1 was directly isolated from pUC-gp120-NSS by *Bgl*II digestion. To insert these *Bgl*II fragments at the 3'-terminus of the HIV-2 gag gene without the protease sequences, two *Bgl*II sites were created by crossover linker mutagenesis, one downstream of the *Pst*I site and another at the C-terminus just upstream of the stop codon. *Bgl*II fragments carrying either V3 or V3 + CD4BD from HIV-1 or HIV-2 were inserted into either the middle of the 3'terminal *Bgl*II site. A total of six constructs were made. The dot-hatched box and solid black box represent the V3 and CD4BD of HIV-1 gp120, respectively. The checkered box and the box with vertical lines represent the V3 and V3+CD4BD of HIV-2 gp120 respectively. The number of amino acids presented in each fragment is indicated.

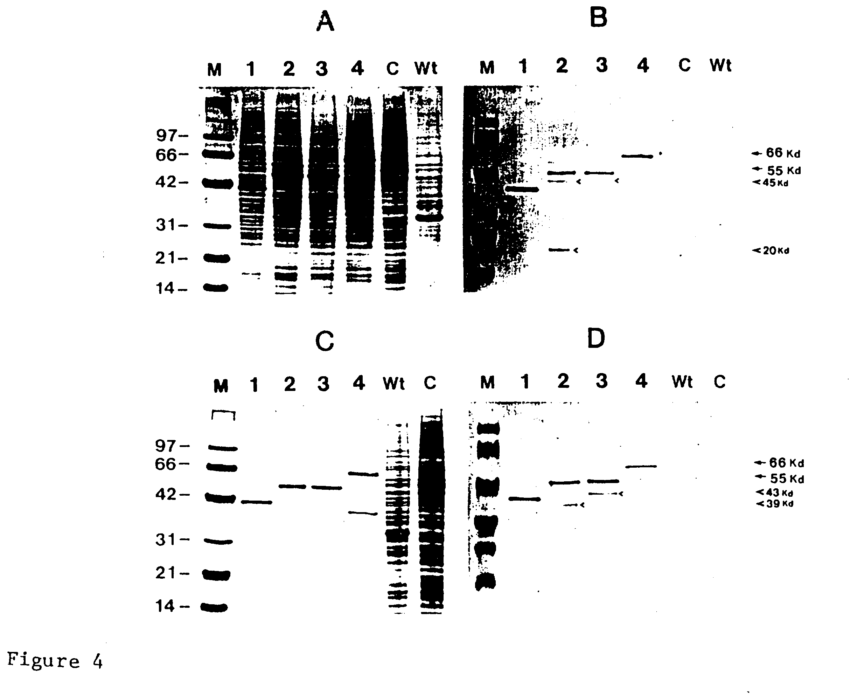

Fig. 4 shows expression of chimeric *gag-env* proteins in SF9 cells infected by recombinant baculoviruses. Recombinant baculovirus infected SF9 cell lysates were analyzed by SDS-PAGE and proteins were stained with Coomassie blue(A) or detected by Western blots with pooled sera from AIDS pationts(B), Lanes 1-4: recombinant viruses AcNPV-HIV-2*gag*, Ac-*gag*M-1V3, Ac-*gag*C-1V3, and Ac-*gag*C-1V3 + 1CD4. The chimeric *gag-env* particles released into the culture supernatant were purified by centrifugation in 20-60% discontinuous sucrese density gradients, subjected to SDS-PAGE and detected by Coomassie blue staining(C) and Western blot(D). Lane 1: Purified *gag* particle; Lanes 2-4 : Purified *ga*gC-1V3, *gag*C-2V3 and *gag*C-2V3+2CD4 chimeric particles; M, Marker proteins; C, uninfected cell control; Wt, wild-type AcNPV-infected cells. The major fusion protein P55 and P66 are indicated with arrows and possible cleavage products are indicated by open arrows.

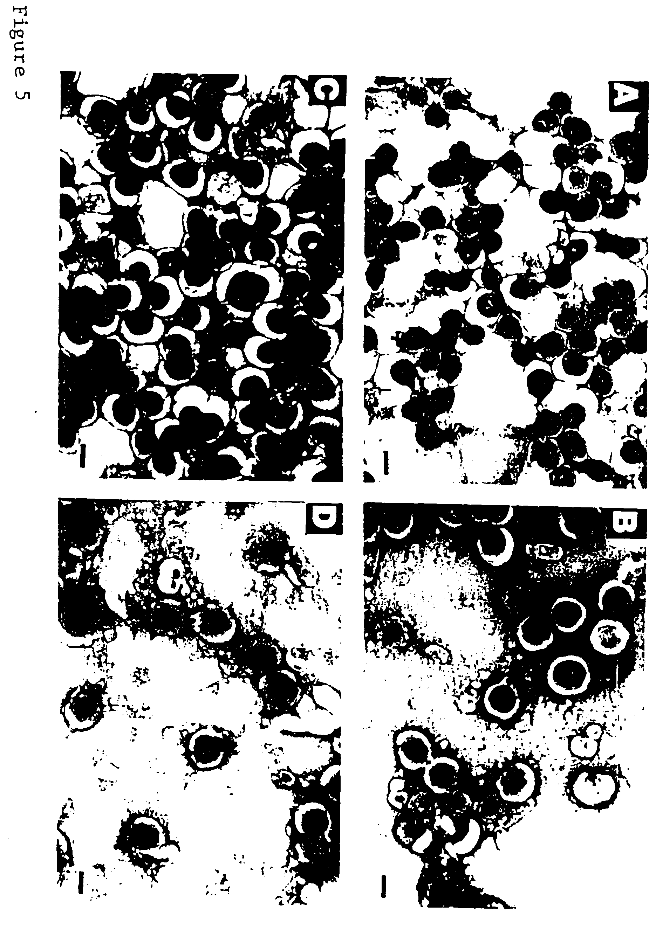

Fig. 5 shows electron micrographs of sucrose gradient-purified *gag-env* chimeric particles. (A) HIV-2 *gag* particles produced by SF9 cells infected with recombinant AcNPV-HIV-2 *gag*, (B) Chimeric *gag-env* particles produced by recombinant Ac-*gag*C-1V3. (C) Chimeric *gag* particles produced by recombinant Ac-*gag*C-2V3, and (D) Chimeric *gag* particles produced by recombinant Ac-*gag*C-2V3+2CD4. Samples were stained with uranyl acetate. The bar represents 100 nm.

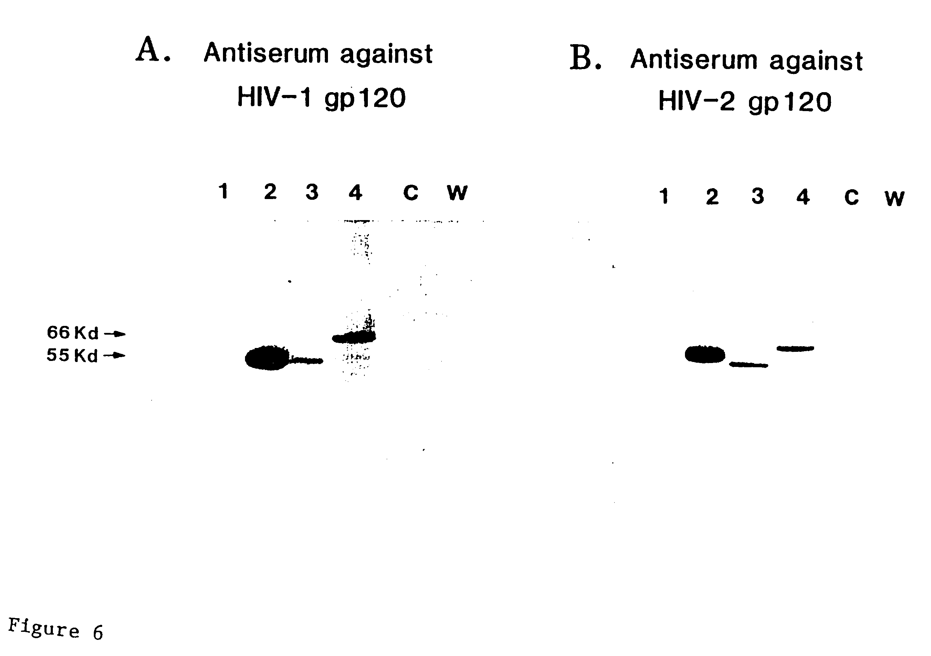

Fig. 6 shows immunoblot analysis of chimeric *gag-env* proteins. The chimeric *gag-env* proteins and gp120 of HIV-1 and HIV-2 were subjected to SDS-PAGE and electro-transferred to nitrocellulose filters. Filters were incubated with rabbit antisera specific for HIV-1 gp120 (a) and HIV-2 (B) and with ¹²⁵I-labeled protein A. Lane 1: HIV-2 *gag* protein; lane 2, gp120 protein; lane 3, chimeric *gag*C-1V3 protein; lane 2, gp120 protein; lane 3, chimeric *gag*C-1V3 protein; lane 4, chimeric *gag*C-1V3+1CD4 protein; C, cell control; W, wild type AcNPV-infected cell control. The rabbit antisera against HIV-1 and HIV-2 gp120 have been described elsewhere.

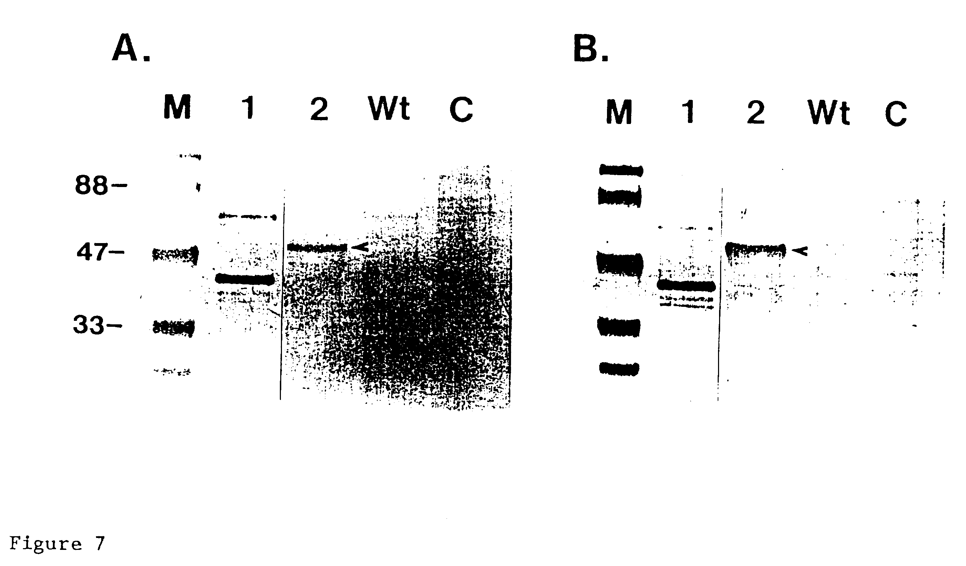

Fig. 7 Western blot analysis using rabbit antisera made against chimeric *gag-env* particles. (A) Anti-*gag*C-1V3 serum recognized non-glycosylated gp120 protein of HIV-1. Lane 1, HIV-2 *gag* protein; Lane 2, non-glycosylated gp120 protein of HIV-1. (B) Anti-*gag*C-2V3 serum recognized non-glycosylated gp120 protein of HIV-2. Lane 1, HIV-2 *gag* protein; lane 2, non-glycosylated gp120 protein of HIV-2. Wt, wild-type AcNPV-infected cells at day 3 postinfection(p.i.). C, cell control. Sera were diluted 1:200 and the Bio-Rad immuno-Blot AK detection system was employed.

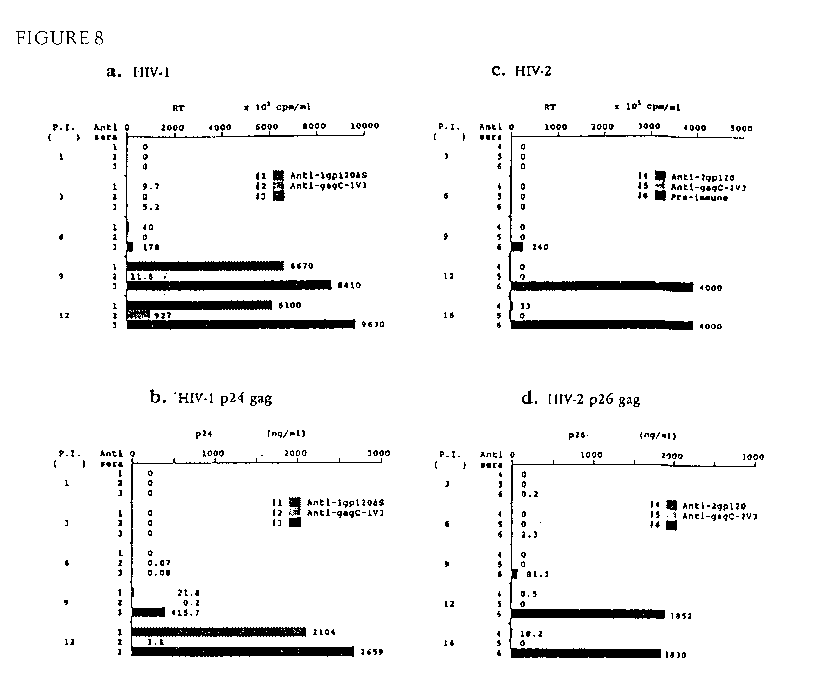

Fig. 8 shows neutralization of HIV-1_{IIIB} and HIV-2_{ROD} infection with immune rabbit sera. Antisera against *gag*C-1V3 and *gag*C-2V3(HIV-2) chimeric particles were diluted and tested for neutralization of virus using reverse transcriptase and viral p24 or p 26 assays. a and b: HIV-1; and d: HIV-2. Pre-immune serum( 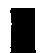 ) ; V3 specific immune serum ( 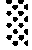 ) from rabbits immunized with chimeric *gag*-V3 particles of HIV-1 or HIV-2; anti-gp120 sera( 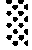 ) specific for gp120 of HIV-1 OR HIV-2. The neutralizing activity of anti-*gag*C-1V3 and anti-*gag*C-2V3 sera were determined by incubation of sera(1:5 dilution) with stock virus preparation of HIV-1_{IIIB}(5000 TCID₅₀) or HIV-2_{ROD} (8000 TCID₅₀) at 37°C for 1 hour before infecting H9 cell. Viral infection was monitored by reverse transcriptase activity(a and c) and the production of HIV-1 p24(b) or HIV-2 p26(d) *gag* proteins at 1-16 days p.i.

Other objects, features and advantages of the invention will be hereinafter become more readily apparent fromthe following description.

### DETAINLED DESCRIPTION OF THE PREFERRED EMBODIMENTS

The present invention provides the *gag* chimeric protein of HIV, which retain both antigenic and immunogenic properties of *gag* and a portion of *env* proteins.

The present inventors reported previously that expression of the *gag* coding sequences of HIV-2, lacking the protease gene, in insect cells produced virus-like particles(Virology 179, 874-880, 1990).

The C-terminus of the gag protein, including the zinc finger domain, is not necessary for particle formation(Fig. 1). Therefore, it is possible to replace the C-terminus of the *gag* precursor protein with other sequences without losing the ability of the gag protein to form virus-like particles. The *gag* protein has the unique ability to form particles in the absence of all other components of the virus, and the chimeric *gag* particles are devoid of genomic RNA. Formation of chimeric *gag* particles containing the major neutralizing epitope(V3) and/or the CD4-binding domain(CD4BD) of gp120 may allow efficient generation of HIV-neutralizing antibodies; antigens presented in a particulate form may enhance immunogenicity of the epitopes and multiple copies of specific epitopes can be presented. Furthermore, secreted chimeric particles can be safely and easily collected and purified from cell culture media by centrifugation.

In this application, we show mapping of essential domains of *gag* protein for particle formation and construction of six different chimeric *gag* genes containing either the V3 loop(V3) or the V3 loop plus the CD4 binding domain(V3 + CD4BD) of gp120 from HIV-1 or HIV-2. These constructs were expressed in insect cells using a baculovirus expression vector. The minimum length of HIV-2 gag to form particles is 376 amino acids and 373rd position proline is essential for the particle formation. The chimeric gene construction reveals that only certain combinations of fusion proteins are expressed, assembled as virus-like particles, and retain antigenicity and immunogenicity of both *gag* and *env* epitopes.

The present invention provides a map of the minimum length of HIV-2 *gag* gene sequences which are required for the gag particle formations using deletion mutagenesis.

The amino acids which is deleted up to 143 amino acids at the C-terminus from the 519 amino acid sequence, which retains 376 amino acids at the N-terminus is the minimum protein for the gag particle formation of HIV-2. In contrast, deletion of four more amino acids, which retain 372 amino acids at the N-terminus, abolished the particle formation.

Site-directed mutagenesis revealed that the proline at amino acid position 373rd, but not 375th and 377th, is essential for the particle formation.

To map the *gag* protein domain(s) which is essential for the particle formation, both deletion and site-directed mutagenesis were carried out. Fig. 1 clearly demonstrates that 376 amino acids at N-terminus are required for the particle formation. In contrast to previous reports, the zinc-finger domain at the C-terminus is not required for the gag particle formation. Furthermore, the present site-specific mutants showed that the 373rd position proline is essential for the particle formation as shown in Fig. 2. The results in Fig. 1 and Fig. 2 indicate that one must retain uninterrupted 1,128 base paris of HIV-2 gag reading frame is essential to package foreign epitopes into *gag* particles.

The present inventors have constructed six different combinations of chimeric genes by coupling the truncated HIV-2 *gag* gene which will code for 425 amino acids to the neutralizing domain(V3) or neutralizing and the CD4 binding domains(V3+CD4BD) of gp 120 *env* gene sequences from HIV-1 or HIV-2. Such a preparation has led to the completion of the present invention.

The *env* gene sequences were either inserted into the middle of the *gag* gene or at the 3' terminus of the *gag* gene. Virus-like particles were formed by chimeric gene products only when the env gene sequences were linked to the 3' terminus of the *gag* gene. Insertion of *env* gene sequence in the middle of the *gag* gene resulted in high level chimeric gene expression but without the formation of virus-like particles.

Especially, three different chimeric proteins: (1) *gag*(425 amino acids) with HIV-1 V3(91 amino acids), (2) *gag* with HIV-2 V3(90 amino acids) and (3) *gag* with HIV-2 V3 + CD4BD(198 amino acids) formed virus-like particles that were secreted into the cell culture medium(cf. Table 1).

**TABLE 1**

| Comparison of particle formation and yield of chimeric fusion proteins | | | |
|---|---|---|---|
| Recombinant Viruses | Particle formation* | Sucrose concentration** | Yield/5x10⁸cell /liter |
| AcNPV-HIV-2*gag* | + | 40% | 30mg |
| Ac-*gag*M-1V3 | - | - | - |
| Ac-*gag*C-1V3 | + | 50% | 6mg |
| Ac-*gag*M-2V3 | - | - | - |
| Ac-*gag*C-2V-3 | + | 50% | 25mg |
| Ac-*gag*C-2V3+2CD4BD | + | 60% | 2mg |

| | | | |
|---|---|---|---|
| note) * Plus sign denotes virus-like particles were recovered from the cell culture media. | | | |
| ** Virus-like particles were banded on top of these sucrose solutions after ultracentrifugation. | | | |

Accordingly, the present invention provides a recombinant baculovirus containing chimeric gene.

Recombinant baculovirus of the present invention can be prepared by using *Autographa Californica nuclear polyhedrosis* virus(AcNPV) or *Bombyx iridexcent* virus.

The recombinant virus is obtained by inserting the chimeric gene into AcNPV genome and purified by the consecutive plaque assays to select polyhedrin negative recombinant virus. The AcNPV inserted with gag of HIV-2 is deposited with the ATCC as a deposit number VR2314, AcNPV inserted with gag of HIV-2 and V3 of HIV-1 as a number VR2316, AcNPV inserted with gag of HIV-2 and V3 of HIV-2 as a number VR2317, on February 26, 1991 respectively. These are guaranteed for furnishing for the purpose of research.

The insect cells selected from the group consisted of *Spodoptera frugiperda* cells, *Mamestra brassica* cells, *Trichoplusia ni* cells, *Bombyx mori* cells and *Bombyx mori* silkworm cells were infected with recombinant baculovirus obtained as above to make expression of *gag* chimeric protein.

The chimeric *gag* particles of the invention elicit neutralizing antibodies in the rabbits which completely block HIV infection.

The chimeric *gag* particles of the invention can be used for the antigen of AIDS vaccine or of diagnostic reagent. The AIDS vaccine according to the present invention exhibits specific immune reaction against immunogen including vaccine used at pre or post exposure for prevention from infection of HIV, or used for immunotherapeutic treatment.

The vaccine of the invention may include the conventional absorbent, stabilizer, adjuvant, carrier etc. and it may be administered through the conventional route.

Other features of the invention will become apparent in the course of the following description of the exemplary embodiments which are given for illustration of the invention and are not intended to be limiting thereof.

### EXAMPLES

### Example 1 <Preparation of plasmids>

*Autographa californica nuclear polyhedrosis* virus(AcNPV) and recombinant AcNPV were grown and assayed in *Spodoptera frugiperda*(SF9) cell monolayers using complete TNM-FH medium containing 10% fetal bovine serum at 27°C as described in Adv. Virus Res. 35, 177-192, 1988. Wild type AcNPV DNA was purified by the method of Smith and Summers(Virology 89, 517-527, 1978).

Plasmids pHXB-2D and p1BM containing the entire HIV-1_{HXB2D} and HIV-2_{NIHZ} genomes respectively were obtained from Dr. R. Gallo(National Institutes of Health, Bethesda, MD). The recombinant plasmids pUC19-gp120-NSS, pUC18-gp120 and pUC19-GAG containing full-length cDNA copies of the HIV-1 *env*, HIV-2 *env* and a truncated *gag* gene of HIV-2(which codes 425 amino acids) respectively were subcloned from pHXB-2D and p1BM as described by the inventors in Virology 179, 874-880, 1990.

### Example 2 <Preparation of chimeric gene>

To subclone the V3 and V3 + CD4B of the HIV-1 and HIV-2 *env* genes into pUC19-GAG recombinant plasmid pUC19-gp120-NSS and pUC19-gp120 were used as a templates and the specific regions were amplified with appropriate oligonucleotide primers. That is to say, plasmids pUC19-gp120-NSS(HIV-1) and pUC18-gp120(HIV-2) were used as templates for polymerase chain reaction(PCR) amplification of an HIV-1 DNA fragment corresponding to V3 domain(91 amino acids, position 273-363) and HIV-2 DNA fragments corresponding to V3 equivalent domain(90 amino acids, position 294-383) and V3 + CD4BD(19 amino acids, position 294-491). All primers were designed to create a *Bgl*II restriction site at the 5' end so that gp120 DNA fragments could be inserted into the *Bgl*II site of pUC19-GAG.

The HIV-1 V3 DNA fragment was amplified using L-1: (5'-CGAAGATCTGTCAATTTCACGG-3') and L-2(5'-GCAGATCTTTGCTTAAAGATTA-3') primers, which are complementary to nucleotides 816 through 836 and 1075 through 1089 respectively. The V3+CD4BD DNA fragment of HIV-1 was generated from pUC19-gp120-NSS *Bgl*II digestion at nucleotide positions 816 and 1398 of gp120. Primers L-3(5'-CGAGATCTCATTGTTAAGAGGCC-3') and L-4 (5'-GCAGATCTTCTGCAGTTAGTCC-3'), complementary to nucleotides position 879 through 893 and 1135 through 1149 of HIV-2 gp 120 respectively, were used to synthesize the HIV-2 V3 DNA fragment. Primers L-3 and L-5 (5'-GCAGATCTCTTTACTGATGTAG-3'), which is complementary to nucleotides 1459 through 1473 of HIV-2 gp120 were used to synthesize the HIV-2 V3+CD4BD DNA fragment. PCR was performed according to the procedures provided with the Geneamp kit(Norwalk, CT). Briefly, 20 ng of linearized gp120 DNA was added to 100 µl PCR reaction mixture(10mM Tris-HCl, 50mM KCl, 1.5 mM MgCl₂ and 0.01% gelatin) containing 20 µM each dNTP, 2.5 Units of Taq polymerase, and 20 µM of each oligonucleotide primer.

The V3 and V3+CD4BD genes were amplified for 30 PCR cycles(94°C for 1 min., 45°C for 3 min.). The chimeric *gag* gene constructs containing the V3 and V3+CD4BD fragments were confirmed by dideoxy DNA sequencing of double stranded DNA, using the Sequence Kit(trademark of United States Biochemical Corporation).

*gag*M-1V3, *gag*C-1V3, *gag*C-1V3+1CD4BD, *gag*M-2V3, *gag*C-2V3, *gag*C-2V3+2CD4BD chimeric genes were prepared by the above method. The numbers 1 and 2 in front of V3 and CD4BD denote genes from HIV-1 and HIV-2 respectively, gagM denotes the chimeric gene inserted into the middle of the *gag* gene, and *gag*C denotes the chimeric gene inserted into the terminus of the *gag* gene. The procedure for generation of the present chimeric gene showed briefly in Fig. 3.

### Example 3 <Production of Recombinant Baculovirus>

The chimeric genes obtained from the Example 3 were isolated after digestion with *Bam*HI and inserted into the baculovirus transfer vector pAcYM1. SF9 cells were co-transfected with mixtures of infectious wild type AcNPV DNA(1 µg) and recombinant plasmid DNAs(4 µg) using the standard procedure(Adv. Virus Res., 35, 177-192, 1988).

Among the recombinant baculoviruses obtained from the above procedure, AcNPV carrying *gag* of HIV-2(hereinafter called 'AcHIV2GAGYK')was deposited as a deposit number VR2314, AcNPV carrying *gag* of HIV-2 and V3 of HIV-1 (hereinafter called 'AcHIV2GAG+1V3YK') as a deposit number VR2316, and AcNPV carrying *gag* of HIV-2 and V3 of HIV-2(hereinafter called 'AcHIV2GAG+2V3YK') as a deposit number VR2317 with the American Type Culture Collection(ATCC) on February 26, 1991 and these are guaranteed for furnishing of samples for the purpose of research.

After incubation of recombinant baculovirus at 27°C for 4 days, culture supernatants were harvested and titrated on 80% confluent monolayers of SF9 cells. To obtain polyhedrin-negative recombinant AcNPV, the plaques lacking polyhedra were picked, purified by three consecutive plaque assays, and used to produce virus stock of 2x10⁸ PFU/ml.

### Example 4 <Expression of Chimeric particles>

SF9 cells were infected with either wild type AcNPV or recombinant AcNPV carrying chimeric genes at multiplicities of infection of 5 PFU/cell and incubated at 27°C. After appropriate incubation times(usually 3 to 4 days), cells were harvested and washed twice with PBS. Whole cell lysates were prepared by resuspending the cell pellet in water and adding an equal volume of 2x dissociation buffer(10% beta-mercaptoethanol, 10% SDS, 25% glycerol, 100 mM Tril-HCl, pH 7.0, 0.04% bromophenol blue). Cell lysates were analyzed by electrophoresis in 12% polyacrylamide gel containing SDS(SDS-PAGE) and the protein bands were visualized by staining with Coomassie blue(Nature 227, 283-303, 1987).

Western blot analyses were then performed using sera from AIDS patients and the Bio-Rad Immune Blot AK system. The results showed in Fig. 4.

Fig.4A shows the proteins produced by recombinant baculoviruses Ac-*gag*M-1V3, Ac-*gag*C-1V3 and Ac-*gag*C-1V3+1CD4BD. Since the original HIV-2 *gag* protein has 93 amino acids deleted from the C-terminus, an insertion of V3(91 amino acids) or V3+CD4BD(194 amino acids) of HIV-1 gp120 into the *gag* gene would be expected to produce proteins of 55 KDa and 66 KDa, respectively.

As shown in Fig.4A, strongly stained protein bands migrating at either 55 KDa or 66 KDa were observed in the lysates of SF9 cells infected with Ac-*gag*M-1V3(lane 2), Ac-*gag*C-1V3(lane 3) and AC-*gag*C-1V3+1CD4BD(lane 4) but were not present in lysates of mock or wild type baculovirus-infected cells.

Western blot analysis revealed that both the p55 and p66 fusion proteins were recognized by pooled HIV-1 positive human sera(Fig.4B, lane 2, 3, and 4). No specific reaction was observed with mock- and wild type AcNPV-infected cells.

The results clearly demonstrate that insertion of either HIV-1 V3 or V3+CD4BD into the *gag* protein resulted in expression of proteins at levels at least as high as that of recombinant AcNPV-HIV-2*gag* alone(Fig.4A, lane 1).

Similar results were obtained when three other recombinant baculoviruses containing the V3 and V3+CD4BD genes from HIV-2 gp120, Ac-*gag*M-2V3, Ac-*gag*C-2V3, and Ac-*gag*C-2V3+2CD4BD were analyzed.

### Example 5 <Purification of Chimeric particles>

The cell culture fluids obtained from the Example 5 was collected after centrifugation at 1,000 x g for 20 min. Chimeric particles in the culture supernatant were collected by ultracentrifugation in a Beckman SW 28 rotor at 80,000 x g for 1 hr and resuspended in PBS containing 0.1% Tween 20, 10 µg/ml aprotinin and stored at 4°C. A band containing *gag* particle was collected from a 20-60% discontinuous sucrose gradient, diluted at least 10-fold with PBS and pelleted in an SW28 rotor at 80,000 x g for 1 hour. The pellet was gently suspended in PBS. *gag*C-1V3 and *gag*C-2V3 particles were recovered from the 50% sucrose cushion, whereas *gag*C-2V3+2CD4BD chimeric particles banded on top of the 60% sucrose cushion. The results of the morphology of sucrose gradient purified particles by transmission electron microscopy using uranyl acetate staining was shown in Fig. 5.

HIV-2 gag particle were spherical (Fig.5A), had diameters of approximately 100 nm, and were similar to those of mature HIV-1 particles budding from HIV-1 infected cells. The chimeric particles *gag*C-1V3(Fig. 5B), *gag*C-2V3(Fig. 5C) and *gag*C-2V3+2CD4BD(Fig. 5D) exhibited morphologies similar to the *gag* particles. The peripheral granular material frequently showed striations with a periodicity suggesting a helical arrangement. It formed a shell-like layer probably inside a lipid membrane and was presumably rigid. The only difference between the *gag* particles and chimeric *gag* particles was that the chimeric *gag* particles are slightly larger with approximate diameters of 130 nm, similar to the diameter of mature HIV-1 particles.

### Experimental Example 1 <Antigenity of Chimeric gag Particles>

Antigenicity of chimeric *gag* particles was investigated by immunoblot analysis. Purified chimeric *gag* particles were subjected to SDS-PAGE and analyzed by Western blotting with rabbit antisera directed against HIV-1 and HIV-2 gp120 and ¹²⁵I-labeled protein A. The results are shown in Fig. 6. In this Fig. 6, lane 1 is HIV-2 *gag* protein; lane 2, gp120 protein; lane 3, chimeric *gag*C-1V3 protein; lane 4, chimeric *gag*C-1V3+1CD4 protein; C, cell control; and W is wild type AcNPV-infected cell control.

The HIV-2 *gag* protein was not recognized by anti-gp120 sera(Fig. 6A and B, lane 1) while the nonglycosylated forms of gp120 of HIV-1 and HIV-2 showed strong reactivity with their corresponding antisera(Fig.6A and B, lane 2). The 55 KDa and 66 KDa fusion proteins were specifically recognized by rabbit antisera against HIV-1 or HIV-2 gp120s(Fig.6A and B, lanes 3 and 4). The results clearly demonstrate that the chimeric proteins can be detected by antiserum specific for gp120 and reaffirm that inserted sequences of V3 or V3+CD4BD are antigenic.

### Experimental Example 2 <Immunogenicity of Chimeric gag-V3 Particles>

In order to examine the capacity of particles to induce antibodies to both *gag* and *env* proteins of HIV, rabbits were immunized four times at 4 week intervals with purified *gag*C-1V3 and *gag*C-2V3 chimeric particles. The immune rabbit sera were collected 2 weeks after the last immunization and tested for their ability to recognize gp120 of HIV-1 and HIV-2.

As shown in Fig. 7, the antisera made against both *gag*C-1V3 and *gag*C-2V3 chimeric particles recognize not only carrier HIV-2 *gag* protein(Fig.7A, B, lanes 1) but also non-glycosylated gp 120 of HIV-1(Fig. 7A, lane 2) and HIV-2(Fig.7B, lane 2).

In contrast, neither wild type AcNPV infected SF9 cells nor uninfected SF9 cells contain proteins which were recognized by the antisera. These results clearly demonstrate that the V3 loop domain in chimeric gagC-1V3 and gagC-2V3 particles retain both antigenic and immunogenic properties.

### Experimental Example 3 <Immune Sera Against Chimeric gag-V3 Particles of HIV-1 or HIV-2 Neutralize Virus Infectivity in Vitro>

Rabbit anti-sera directed agaginst chimeric particles were used to neutralize the infectivity of HIV as assayed by reverse transcriptase(RT) activity and *gag* p24 production. Rabbits were immunized four times at one month intervals with intramuscular injections of 25 µg of the density gradient-purified chimeri*c gag* particles. Rabbit anti-*gag*C-1V3 and anti-*gag*C-2V3 sera(25 µl) were mixed with 100 µl of virus which represented 5000 TCID₅₀ of HIV-1_{IIIB} or 8000 TCID₅₀ of HIV-2_{ROD}, respectively and the mixtures were used to infect H9 cells. The amount of p24 *gag* protein and reverse transcriptase(RT) activity in the culture media were assayed as quantitation of virus production on different days after infection (days 1-16) and the levels were compared with those of control samples in which virus was incubated with preimmune sera or rabbit anti-gp120 serum.

Fig. 8 shows that both rabbit anti-*gag*C-1V3 and anti-*gag*C-2V3 sera contained antibodies capable of neutralizing HIV infection of H9 cells. By day 9 postinfection, antisera to *gag*C-1V3 and *gag*C-2V3 chimeric particles completely blocked the production of HIV-1 and HIV-2, respectively. However, at day 12 post-infection, a small amount of HIV-1 was detected in cultures treated with anti-*gag*C-1V3 serum(Fig. 8, a and b). In contrast, the antisera to *gag*C-2V3 chimeric particles completely neutralized HIV-2 infectivity(Fig. 8, c and d). No reduction in RT and p24 *gag* protein production were observed with pre-immune sera. Rabbit anti-gp120 sera of HIV-2 showed stronger neutralizing activity of HIV-1_{ROD} than rabbit anti-gp120 sera of HIV-1 against HIV-1_{IIIB}(Fig. 8).

From the above, it is recognized that the chimeric protein according to the present invention showed excellent neutralizing activity against HIV-1 and HIV-2.

## Claims

1. A recombinant gag-env chimeric polypeptide particle of human immunodeficiency virus, comprising: an HIV-2 gag polypeptide selected from the group consisting of HIV-2 gag polypeptides which extend from the N-terminal amino acid of gag to a minimum of amino acid 376 and a maximum of amino acid 425 and wherein said gag polypeptides have a proline at amino acid 373; and an env polypeptide from HIV comprising the V3 loop domain of gp 120; the env polypeptide linked to the C-terminus of the gag polypeptide.

2. The gag-env chimeric polypeptide particle according to Claim 1, wherein the env polypeptide comprises the V3 loop domain of gp 120 and the CD4 binding site.

3. The gag-env chimeric polypeptide particle according to Claim 2, wherein the gag-env chimeric polypeptide is expressed in an insect cell by a recombinant baculovirus comprising a chimeric HIV gag-env gene encoding the gag-env chimeric polypeptide of Claim 1.

4. The gag-env chimeric polypeptide particle according to Claim 4, wherein the recombinat baculovirus is ATCC deposit number VR2316 or VR2317.

5. A vaccine composition comprising the recombinant gag-env chimeric polypeptide particle of HIV according to any one of the Claims 1 to 4, together with a pharmaceutically acceptable carrier therefor.

6. A vaccine composition according to Claim 5, in which the gag-env chimeric protein is one expressed from the recombinant baculovirus of ATCC VR2316 or ATCC VR2317.

## Patentansprüche

1. Rekombinantes chimäres gag-env-Polypeptidpartikel von menschlichem Immunschwächevirus, umfassend: ein HIV-2-gag-Polypeptid, das aus der Gruppe ausgewählt ist, die aus HIV-2-gag-Polypeptiden besteht, die sich von der N-terminalen Aminosäure von gag mindestens bis Aminosäure 376 und höchstens bis Aminosäure 425 erstrecken, wobei die gag-Polypeptide ein Prolin an Aminosäureposition 373 aufweisen; und ein env-Polypeptid von HIV, das die V3-Schleifendomäne von gp 120 umfaßt; wobei das env-Polypeptid mit dem C-Terminus des gag-Polypeptids verknüpft ist.

2. Chimäres gag-env-Polypeptidpartikel nach Anspruch 1, wobei das env-Polypeptid die V3-Schleifendomäne von gp 120 und die CD4-Bindungsstelle umfaßt.

3. Chimäres gag-env-Polypeptidpartikel nach Anspruch 2, wobei das chimäre gag-env-Polypeptid in einer Insektenzelle durch ein rekombinantes Baculovirus, das ein chimäres HIV-gag-env-Gen, das für das chimäre gag-env-Polypeptid von Anspruch 1 kodiert, umfaßt, exprimiert wird.

4. Chimäres gag-env-Polypeptidpartikel nach Anspruch 4, wobei es sich bei dem rekombinanten Baculovirus um die ATCC-Hinterlegung Nr. VR2316 oder VR2317 handelt.

5. Vaccinzusammensetzung, die das rekombinante chimäre gag-env-Polypeptidpartikel von HIV nach einem der Ansprüche 1 bis 4 zusammen mit einem pharmazeutisch verträglichen Träger dafür umfaßt.

6. Vaccinzusammensetzung nach Anspruch 5, wobei das chimäre gag-env-Protein ein Protein ist, das von dem rekombinanten Baculovirus ATCC VR2316 oder ATCC VR2317 exprimiert wird.

## Revendications

1. Particule polypeptidique chimère recombinante *gag-env* du virus de l'immunodéficience humaine, comprenant : un polypeptide *gag* du VIH-2 choisi dans le groupe constitué par les polypeptides *gag* du VIH-2 qui s'étendent depuis l'acide aminé N-terminal de *gag* jusqu'au minimum l'acide aminé 376, et, au maximum, l'acide aminé 425, et dans laquelle lesdits polypeptides *gag* comportent une proline en position 373 d'acide aminé ; et un polypeptide *env* du VIH comprenant le domaine de la boucle V3 de gp120 ; le polypeptide *env* étant lié à l'extrémité C-terminale du polypeptide *gag.*

2. Particule polypeptidique chimère *gag-env* selon la revendication 1, dans laquelle le polypeptide *env* comprend le domaine de la boucle V3 de gp120 et le site de liaison CD4.

3. Particule polypeptidique chimère *gag-env* selon la revendication 2, dans laquelle le polypeptide chimère *gag-env* est exprimé dans une cellule d'insecte par un baculovirus recombinant comprenant un gène chimère *gag-env* du VIH qui est codant pour le polypeptide chimère *gag-env* de la revendication 1.

4. Particule polypeptidique chimère *gag-env* selon la revendication 3, dans laquelle le baculovirus recombinant porte le numéro de dépôt à l'ATCC VR2316 ou VR2317.

5. Composition vaccinale comprenant la particule polypeptidique chimère recombinante *gag-env* du VIH selon l'une quelconque des revendications 1 à 4, et un véhicule acceptable au plan pharmaceutique pour celle-ci.

6. Composition vaccinale selon la revendication 5, dans laquelle la protéine chimère *gag-env* est une protéine exprimée par le baculovirus recombinant dont le numéro de dépôt à l'ATCC est VR2316 ou VR2317.
